# EUROPEAN PATENT SPECIFICATION

(11) **EP 1 831 114 B1**
(45) Date of publication and mention of the grant of the patent: **25.03.2015**
(21) Application number: 04816673.0
(22) Date of filing: 28.12.2004
(51) Int. Cl.: C02F 3/34, C02F 103/32, C12N 1/20, C12R 1/01

(54) **BIOTECHNOLOGICAL PROCESS FOR NEUTRALIZING ALKALINE BEVERAGE INDUSTRIAL WASTE WATER**
BIOTECHNOLOGISCHES VERFAHREN ZUR NEUTRALISATION VON ALKALISCHEM ABWASSER DER GETRÄNKEINDUSTRIE
PROCÉDÉ BIOTECHNOLOGIQUE POUR LA NEUTRALISATION D'EAUX USÉES ALCALINES DE L'INDUSTRIE DES BOISSONS

(43) Date of publication of application: 12.09.2007
(73) Proprietor: Council of Scientific and Industrial Research, New Delhi 110 001 (IN)
(72) Inventor: KUMAR, Rita Inst. of Genomics and Integrative Biol., Delhi 110 007 (IN); KUMAR, Anil Inst. of Genomics and Integrative Biol., Delhi 110 007 (IN)
(74) Representative: Fairbairn, Angus Chisholm
(86) International application number: PCT/IN2004/000421
(87) International publication number: WO 2006/070388

(56) References cited:
- US-A1- 2002 064 864
- ISAO YUMOTO ET AL: "Exiguobacterium oxidotolerans sp. nov., a novel alkaliphile exhibiting high catalase activity" INTERNATIONAL JOURNAL OF SYSTEMATIC AND EVOLUTIONARY MICROBIOLOGY, no. 54, 23 April 2004 (2004-04-23), pages 2013-2017, XP002347351 GBSOCIETY FOR GENERAL MICROBIOLOGY, READING,
- FRUEHLING A ET AL: "EXIGUOBACTERIUM UNDAE SP. NOV. AND EXIGUOBACTERIUM ANTARCTICUM SP. NOV" INTERNATIONAL JOURNAL OF SYSTEMATIC AND EVOLUTIONARY MICROBIOLOGY, SOCIETY FOR GENERAL MICROBIOLOGY, READING,, GB, vol. 52, no. PART 4, 2002, pages 1171-1176, XP009013832 ISSN: 1466-5026
- Z. ULUKANLI: "Alkaliphilic Micro-organisms and Habitats" DOGA. TURK BIYOLOJI DERGISI - TURKISH JOURNAL OF BIOLOGY, no. 26, 2002, pages 181-191, XP002347352 TRANKARA

## Description

### Field of the invention

The present invention relates to a bacterial isolate, *Exiguobacterium sp.* (MTCC 5183) deposited at International Depository IMTECH, Sector 39A, Chandigarh, India recognized by Budapest Treaty. More particularly, the present invention relates to a process of preparing bacterial isolate, *Exiguobacterium sp.,* useful in neutralization of highly alkaline waste water from beverage industry deposited at International Depository IMTECH, Sector 39A, Chandigarh, India recognized by Budapest Treaty.

### Background and prior art

Stringent laws and frequent checks by the authorities reflect environmental concerns now held by society. Thus, for instance, now pH of wastewater of industries such as beverages may only deviate minimally from neutral point when discharged into a receiving watercourse or sewerage system. Various chemicals are available to neutralize the highly alkaline beverage industrial wastewater depending upon the application. In most cases, Sulfuric Acid (H₂SO₄) is used. The end user must consider the concentration to be used, must carefully analyze all the chemistries involved, must review manufacturers' warnings and instructions, and must consider common safety measures for hazardous liquids.

The process of treating wastewaters comprises treating with chemicals which can be either acids or bases or capable of forming acids or bases on addition to wastewater. Various chemicals are available for industrial neutralization depending upon the application and whether neutralization of an acidic or basic solution is being carried out.

The most commonly used neutralization chemicals for acid or base neutralization are 98% Sulfuric acid and 50% Sodium hydroxide. In many cases these are very good choices, however, there are many considerations when selecting chemicals and these may not always be the best selection.

The selection of the chemicals used for the neutralization of an acid or base is almost as important as the design of the neutralization system. Some of the major points to consider in the selection of chemicals are listed below:
- Health and Safety.
- Cost and Convenience.
- Physical Properties of neutralizing chemicals.
- Storage Environment.

An explanation of chemical selection criteria is as follows:

Health and Safety: Mixing of chemicals can lead to extreme hazardous or noxious reactions. For example: addition of any acid to cyanide bearing solution results in the release of deadly HCN gas.

Cost and Convenience: Most acids and bases work in most applications. Sulfuric acid (H₂SO₄), for example, is less costly and more potent than nitric acid. Concentration is also an important consideration in evaluating cost. Sulfuric acid, for example, can be purchased in concentration ranging from near 0% to 98%. Higher concentrations are generally less expensive.

Physical Properties: Physical properties of the selected reagent must be considered carefully. 50% Sodium hydroxide (NaOH), for example, begins to freeze at temperatures below 60°F. Decreasing the concentration to 25% eliminates this concern altogether. Hydrochloric acid (HCl), for example, out gasses severely. The gas is very highly corrosive and will attack all metallic objects. Therefore, if HCl is used it must be properly vented or used outdoors where the gasses can easily dissipate.

Storage Environment: Storage issues such as types of tanks and secondary containment available, familiarity of operators in handling hazardous chemicals, the dangers of refilling storage containers or procedures for transferring from bulk containers are of concern.

The most commonly used neutralizing chemicals are listed below:
- Acids: Sulfuric Acid, Hydrochloric Acid, Nitric Acid, Phosphoric Acid and Carbon Dioxide which forms Carbonic Acid in water
- Bases: Sodium Hydroxide (Caustic Soda), Calcium Hydroxide, Calcium Carbonate (Lime or Limestone), and Ammonium Hydroxide

### Neutralization with Acids

Sulfuric Acid is the most widely used and produced chemical in the world. Available in concentrations ranging from 0% to 98%, sulfuric acid is most economical of all and used universally for neutralization reactions. It is easier and safer to use than HCl or HNO₃ and is more potent than all of the other acids except for phosphoric acid. Sulfuric acid is typically used in concentrations ranging from 25% to 96%. However, 30% to 50% concentrations of sulfuric acid are generally recommended.

Hydrochloric Acid (HCl), also known as muriatic acid, is the second most commonly used acid in industry (sulfuric acid being the first). It is very effective, and relatively inexpensive. At a maximum available concentration of 37%, HCl is about 1/3 as potent as sulfuric acid, thus making it relatively more expensive to use. Depending on temperature and agitation, HCl at concentrations above 10% evolves hydrogen chloride vapors which combine with water vapors present in the air. The gas thus formed is highly corrosive and attacks all metallic objects including building structures, sprinkler heads, copper wiring, stainless steel, etc. Therefore, it must be properly vented or used outdoors where gasses can easily dissipate.

Nitric Acid (HNO₃) though a widely used chemical in many industries it does not enjoy the popularity of hydrochloric or sulfuric acid, as it is more expensive to use than either of them. Nitric acid evolves noxious gas which on combines with water vapors present in the air. The gas is highly corrosive and attacks all metallic objects including building structures, sprinkler heads, copper wiring, stainless steel, etc. Therefore, it must be properly vented or used outdoors where the gasses can easily dissipate.

Phosphoric Acid (H₃PO₄), very widely used in the production of agricultural fertilizers and detergent products it is a relatively inexpensive acid. However it still does not compete well with sulfuric and hydrochloric acid as it is a weak acid and does not fully disassociate in water at normal concentrations. This renders it safer to use compared to sulfuric or hydrochloric acid and the evolution of gasses is rare. It tends to buffer neutralization reactions and this makes for a slower reaction that is easier to control. Due to its cost (as compared to sulfuric acid) and availability, phosphoric acid is not commonly used in neutralization system.

Carbon Dioxide (CO₂), the third most concentrated gas found in earth's atmosphere, CO₂ is itself not an acid. It forms carbonic acid (H₂CO₃) when dissolved in water; and it is this carbonic acid that brings about the neutralization of alkalinity in solution. The most appealing feature of CO₂ is that it will not lower the pH of water below 7.0 (for all practical purposes). Additionally CO₂ is non corrosive; however as it is heavier than air and thus, asphyxiation is a hazard. Carbon dioxide is difficult to use and its use is limited because the gas must be dissolved into solution to be used. This requires the use of a carbonator, or some method to dissolve the gas into solution. Significant out-gassing also occurs, which does not hold a problem unless the process also requires the settling of solids. In cement pouring operations large amounts of alkaline wastewaters are generated. It is an excellent choice for such applications as the site is temporary, the gas is non-hazardous, can be used in-line assuming retention and mixing is considered and is self-buffering so regardless of dosage it will not lower the pH below 7.5-7.0.

### Alkaliphiles

Several microorganisms exhibit more than one pH optimum for growth depending on growth conditions, particularly nutrients, metal ions, and temperature. The term "alkaliphile" is used for microorganisms that grow optimally or very well at pH values above 9. The first paper concerning an alkaline enzyme of alkaliphilic microorganisms was published in 1971. Over the past two decades, our studies have focused on the enzymology, physiology, ecology, taxonomy, molecular biology and genetics of alkaliphilic microorganisms. Industrial applications of these microorganisms have also been investigated extensively and some enzymes, such as alkaline proteases, alkaline amylases and alkaline cellulases, have been put to use on an industrial scale (Horikoshi. K. (1971) Production of alkaline enzymes by alkalophilic microorganisms. Part 1. Alkaline protease produced by Bacillus No, 221. Agric, Biol, Chem. 36,. 1407-1414., Horikoshi, K. and Akiba, T. (1982) Alkalophilic Microorganisms: A New Microbial World. Springer- Verlag, Heidelberg, Tokyo.)

Subsequently, many microbiologists have published numerous papers on alkaliphilic microorganisms in various fields. Cell surface of alkaliphiles can maintain the intracellular pH values neutral in alkaline environments of pH 10-13. In 1995, new host vector systems were developed by using alkaliphilic *Bacillus* C-125 mutants that are alkaline sensitive, and genes responsible for alkaliphily have been investigated (Horikoshi, K. (1991) Microorganisms in Alkaline Environments, Kodansha-VCH, Tokyo, Weinheim, New York, Cambridge, Basel., Kudo, T., Hino, M., Kitada, M. and Horikoshi, K. (1990) DNA sequences required for the alkalophily of Bacillus sp. strain C-125 are located close together on its chromosomal DNA. J. Bacteriol. 172, 7282-7283.). Yumoto Isao et al. (Int. J. Syst. Evol. Microbiol., (2004), 54, 2013-2017) discloses an alkaliphile *Exiguobacterium oxidotolerans sp.,* isoltaed from a drain pool of a fish processing plant, that exhibits high H₂O₂ degrading and catalase activity.

Although alkaliphiles have been used for a number of industrial applications but there is no research publication regarding the neutralization of beverage industrial wastewater using them. Some work on biological neutralization by a mixture of bacteria in the presence of sugars has been considered for patenting and is disclosed in US Patent Application No. 09/160422, titled 'Microbial Composition and a Process Useful for the Neutralization of Alkaline Waste-Water'.

### Objects of the invention

The main object of the invention is to provide a bacterial isolate *Exiguobacterium sp.* MTCC 5183 deposited at International Depository at IMTECH, Sector 39A, Chandigarh, India recognized by Budapest Treaty.

Another object of the invention is to provide a process for preparing a bacterial isolate *Exiguobacterium sp.* MTCC 5183 deposited at International Depository at IMTECH, Sector 39A, Chandigarh, India recognized by Budapest Treaty.

Another object of the invention is to isolate the bacterium *Exiguobacterium sp.* MTCC 5183 deposited at International Depository at IMTECH, Sector 39A, Chandigarh, India recognized by Budapest Treaty which is useful to neutralize the highly alkaline waste water of beverage industry.

### Summary of the Invention

The present invention provides a bacterial isolate, *Exiguobacterium sp.* MTCC 5183 deposited at International Depository at IMTECH, Sector 39A, Chandigarh, India, as recognized by Budapest Treaty from beverage industria waste water. This bacterial strain is capable to bring down the pH of waste water from 12.00-7.00 units within two hours. The neutralization of alkaline beverage industrial waste water by such buiotechnological process is highly effective and economical as compared to conventional neutralization process by chemical means.

Accordingly the present invention provides a bacterial isolate deposited at International Depository at IMTECH, Sector 39A, Chandigarh, India recognized by Budapest Treaty.

The present invention also provides a process for preparing a bacterium isolate of *Exiguobacterium sp.* MTCC 5183 useful in neutralization of alkaline waste water of beverage industry having a pH in the range of 12.00-11.5, deposited at International Depository at IMTECH, Sector 39A, Chandigarh, India recognized by Budapest Treaty, the process comprising the steps of:
(a) enriching activated sludge contaminated with bacteria comprising *Exiguobacterium sp.* MTCC 5183 by providing an autoclaved aqueous extract of dried activated sludge in an alkaline bacillus medium;
(b) culturing the bacteria;
(c) isolating the bacteria by centrifuging the culture obtained from step (b) after attaining the desired growth to obtain a pellet of bacterial cells as the bacterium isolate.

In one embodiment of the invention, the contaminated activated sludge is obtained from the pipe of an effluent treatment plant of a beverage industry located in Gaziabad, India.

In another embodiment of the invention, the enrichment of the sludge is effected for a period of 40-48 hours at 100-120rpm at 33-35°C in ratio of activated sludge to medium in the range of 1:5-1:10.

In another embodiment of the invention, the culturing of the bacteria is carried out in the alkaline bacillus medium and at a pH in the range of 11.00 - 12.00 and at 37 to 34°C.

In another embodiment of the invention, the bacteria is isolated by centrifuging the culture obtained from step (b) after attaining a growth evidenced by an optical density in the range of 1.5-2.0.

In another embodiment of the invention, enrichment of the sludge is carried out by taking 5-7 g of fresh activated sludge in an autoclaved flask containing 100-110 ml autoclaved aqueous extract of dried activated sludge, 50µl Candid B anti fungal and alkaline bacillus medium.

In another embodiment of the invention, the autoclaved aqueous extract of dried activated sludge is prepared by centrifuging a sterilized sludge mixture at about 4000 rpm for about 20 min.

In another embodiment of the invention, the sterilized sludge mixture is prepared by dissolving dried activated sludge in distilled water in ratio ranges between 1:5-1:10 and autoclaving at about 15 psi for about one hour.

In another embodiment of the invention, the alkaline bacillus medium contains peptone, yeast extract, glucose, K₂HPO₄ and Na₂CO₃ in ratio about 1.0:0.5:1.0:0.1:1.0 by W/ V.

In another embodiment of the invention, the ratio between the autoclaved aqueous extract of dried activated sludge and the alkaline bacillus medium is 1:5-1:10.

In another embodiment of the invention, the bacterium is cultured in the alkaline bacillus medium followed by incubation at 32-37°C/80-120 rpm for 8 hours.

In another embodiment of the invention, grown culture is centrifuged after attaining the heavy growth evidenced by optical density in the range of 2 -2.5.

In another embodiment of the invention, the process further comprises the step of (d) dissolving the pellet obtained from step (c) in phosphate buffer.

The present invention also provides a method of neutralizing alkaline wastewater of beverage industry having pH in the range of 12.00-11.5, the method comprising preparing a bacterium isolate of *Exiguobacterium sp.* MTCC 5183 by a process comprising steps (a) to (d) as above, and adding the bacterial pellet obtained from step (d) to the alkaline wastewater.

In one embodiment of the invention, the method comprises adding the bacterial isolate of *Exiguobacterium sp.* MTCC 5183 to the alkaline wastewater.

In another embodiment of the invention, the alkaline wastewater has a pH of 12.0 to 11.5.

In another embodiment of the invention, the bacterial isolate is added to the alkaline wastewater in a ratio ranging from 1:5-1: 10.

### Detailed description of the invention

The present invention provides a bacterial isolate, *Exiguobacterium sp.* MTCC 5183, deposited at International Depository at IMTECH, Sector 39A, Chandigarh, India recognized by Budapest Treaty. The bacterial isolate, *Exiguobacterium sp.* MTCC 5183 deposited at International Depository at IMTECH, Sector 39A, Chandigarh, India recognized by Budapest Treaty, is capable of neutralizing highly alkaline waste water of beverage industry. The bacterial isolate is capable of growth in a medium of pH in the range of 10- 12.00 and is also capable of lowering high pH of 12.0 to 11.5 of beverage industrial waste water to neutral pH (7.5 to 7.00) within very short period of 1-1.5 hours. The bacterial pellet is used to neutralize the high pH (12.0 to 11.5) of beverage industrial waste water to neutral pH (7.5 to 7.00) in ratio ranging from 1 :5-1 :10. The bacterial isolate is isolated from activated sludge of an effluent treatment plant of local beverage industry located in Gaziabad, India. It is observed that the isolate is Gram positive, non-motile, rod shaped and oxidase negative, hydrolyzes starch, produces acids from glycerol, cellobiose, D-mannose, mannitol, methyl α-D-glucoside, amygdalin and arbutin.

The process of preparing the isolate of bacterium *Exiguobacterium sp.* MTCC 5183, comprises the steps of;
(a) enriching the activated sludge contaminated with bacteria by providing a sludge extract in alkaline baccilus medium for the period of 40-48 hours at 100-120rpm at 33-35°C in ratio ranging from 1:5-1:10;
(b) culturing the bacteria by using a said alkaline baccilus medium at pH 11.00 - 12.00 and at 37 to 34 °C;
(c) isolating the said bacteria by centrifuging the culture obtained from step (b) after attaining the heavy growth (O.D. 1.5-2.0) to obtain the pellet of bacterial cell;
(d) dissolving the pellet obtained from step (c) in phosphate buffer;
(e) neutralizing the alkaline wastewater(pH 12.00-11.5) of beverage industry by adding the bacterial pellet obtained from step (d) in waste water.

Enrichment of the sludge from said site is done by taking 5-7 g of fresh activated sludge in an autoclaved flask containing 100-110 mi sludge extract, 50µl Candid B (anti fungal) and alkaline baccilus medium. The sludge extract is prepared by centrifuging the sterilized sludge mixture at about 4000 rpm for about 20 min. The sterilized sludge mixture is prepared by dissolving the activated sludge in distilled water in ratio ranges between 1:5-1:10 and autoclaved it at about 15 psi for about one hour. The alkaline baccilus medium contains peptone, yeast extract, glucose, K₂HPO₄ and Na₂CO₃ in ratio about 1.0:0.5:1.0:0.1:1.0 by by W/V. A mixture of sludge extract and alkaline baccilus medium is used to entrap the maximum bacterial flora of the said site. The ratio between the said sludge extract and the said alkaline baccilus medium is 1:5-1:10. Isolated bacterial isolates are cultured under defined conditions such as media, temperature, pH, carbon source etc.

All the bacterial isolates (total two) are checked for their neutralizing capability to lower the pH of beverage wastewater in a short period of time. Decrease in pH is monitored by pH meter. A bacterium is selected which is capable to bring down the pH of alkaline beverage wastewater in a short period of about 1 hour. The selected bacterium is cultured under defined conditions using alkaline baccilus medium followed by incubation at 32-37 °C/80-120 rpm 8 hour for neutralizing the alkaline beverage industrial wastewater. The grown culture is centrifuged after attaining the heavy growth O.D. (2 -2.5) and then dissolved in phosphate buffer. Neutralization of the highly alkaline wastewater of beverage industry was done by adding the bacterial pellet in wastewater lowering of pH from 12.0-11.5 to 7.5 - 7.0 was observed in 1.5 h to one hour as checked by pH meter.

The bacterium *Exiguobacterium sp.* is used as a whole cell.

The bacterial strain concerned with the present invention is deposited at International Depository at IMTECH, Sector 39A, Chandigarh, India as recognized by Budapest Treaty.

| **S.No.** | **Culture** | | **MTCC ID No.** |
|---|---|---|---|
| 1. | *Exiguobacterium sp.* | (DSM ID 03-501) | MTCC 5183 |

The above-mentioned bacterial strain exhibits a remarkable capability to neutralize highly alkaline beverage industrial wastewater within a short period of one hour under defined conditions. The bacterial strain of the invention has been isolated from six months old activated sludge from the ETP of a local beverage industry. To isolate a potential bacterial isolate, 10g activated sludge from the said site was added in the 500 ml autoclaved flask containing 100 ml activated sludge extract, 100 ml alkaline bacillus medium and 50 ul Candid B (anti-fungal). Alkaline bacillus medium contained 1gm peptone, 0.5gm yeast extract, 1g glucose, 0.1g K₂HPo₄ and 1g Na₂CO₃. Peptone and yeast extract, autoclaved at 15psi while glucose, K₂HPO₄ and Na₂CO₃ were autoclaved at 10 psi. After autoclaving the different ingredients at different psi, all the ingredients are mixed together aseptically. The enrichment flask was kept at 100 rpm for 48 hours at 35°C.

For the preparation of activated sludge extract, 1kg activated sludge was taken and dried at 50°C for 2 hour. 400g of dried activated sludge was dissolved in 960 ml single distilled water and autoclaved at 15 lbs for 1 hour. After autoclaving, the sample was centrifuged at 5000 rpm for 10 minutes. The supernatant (extract) was collected and stored in sterile bottle for the preparation of enrichment flask and further use.

The enriched activated sludge sample was serially diluted in 0.85% saline. 100ul from each respective dilution was spread onto agar petri plates containing activated sludge extract and 50% ABM. AMB contained peptone, yeast extract, glucose, K₂HPo₄, Na₂CO₃ and 2% agar. Peptone and yeast extract were autoclaved at 15psi while glucose, K₂HPo₄ and Na₂CO₃ were autoclaved at 10psi. After autoclaving the different ingredients at different psi, all the ingredients are mixed together aseptically. The plates thus obtained were incubated at 35 ± 2 °C for 24 - 96 hrs in inverted position.

On the basis of colony morphology and color, total 2 bacterial isolates were selected to check their capability for neutralizing the alkaline wastewater. The single isolated colonies were picked and streaked on fresh plates containing the same medium. The above step was repeated till pure colonies were obtained.

To check the neutralizing capability of the two isolated bacteria, 200 ml beverage industrial wastewater of high pH (12.00) was taken in 500ml glass flask at two places and each bacterial growth was added individually. Decrease in pH was monitored by a pH meter.

Out of two, only one isolate was found capable to grow on high pH (12.00) and bring down the pH of wastewater within a short period of 2 hour. This bacterium was identified as *Exiguobacterium sp. (DSM ID 03-501)* and the main characteristic features are:

This bacterium, Exiguobacterium *sp.* MTCC 5183 (DSM ID 03-501), is facultative aerobic in nature, is gram positive, is non-motile, is oxidase negative, shows optimum growth at 35°C and is also capable of growth in a high pH environment of pH 12.0 to 11.5, can hydrolyze starch as well as produce acids from glycerol, cellobiose, D-mannose, mannitol, methyl α -D-glucoside, amygdalin and arbutin.

In a neutralization experiment, beverage industrial wastewater was taken from a local beverage industry. The bacterium, Exiguobacterium sp. MTCC 5183, as screened above, was inoculated in 200 ml ABM. The culture was incubated at 35°C for 8 hour under shaking conditions ( 100-120rpm). After observing the heavy bacterial growth (O.D. = 2), the culture was centrifuged at 7,000rpm at 4°C. The culture pellet was dissolved in 20ml phosphate buffer (0.05M, pH 6.8). This pellet was added in a flask containing 200 ml beverage industrial waste water (pH -12.00). The flask was kept at under shaking conditions (100-120rpm). Decrease in pH was observed just after 2 hour. This bacterium, Exiguobacterium sp. MTCC 5183 (DSM ID 03-501) has been capable to bring down the pH from 12.0 - 11.5 to pH 7.7- 7 within a short period of 1.5 h to one hour. PH was monitored by a ph meter.

The invention further provides a process for the preparation of bacterial growth useful in neutralizing the alkaline wastewater:
a) enriching the activated sludge of the said site using activated sludge extract and AMB to isolate the bacteria having neutralization capability;
b) using the mixture of activated sludge extract and alkaline bacillus medium (1gm peptone, 0.5gm yeast extract, 1g glucose, 0.1g K₂HPo₄ and 1g Na₂CO₃ for 100 ml) to entrap the desired potential bacteria from the said site;
c) culturing the said bacteria isolated from specific site under defined conditions such as media, temperature, pH, carbon source etc.;
d) checking the neutralizing capability of isolated bacterial isolates by inoculating them in alkaline beverage industrial waste water;
e) decrease in pH was monitored by a pH meter;
f) selecting a bacterial isolate which can neutralize alkaline waste water in a short time;
g) culturing the selected bacterium under defined conditions for neutralizing the alkaline beverage industrial wastewater. ABM was used to grow the culture. The culture flask was incubated at 35°C/120rpm 8 hour in order to obtain heavy growth;
h) centrifuging the resulting culture after attaining the heavy growth O.D. (2.00);
i) collecting the bacterial pellet and dissolving in phosphate buffer (0.05M, pH 6.8);
j) neutralizing the highly alkaline wastewater of beverage industry by adding the bacterial pellet in 200 ml wastewater. Lowering of pH from 12.0-11.5 to 7.5 -7.00 was observed in one hour as checked by pH meter.

The following examples are given by way of illustration and therefore should not be construed to limit the scope of the invention.

### Example 1

In an endeavor of exploring alkaliphilic bacteria, strategic isolation was done to entrap the potential bacterial flora from the specific site. Bacterial were isolated from six months old activated sludge from the ETP of a local beverage industry

To isolate a potential bacterial isolate, 5g activated sludge from the said site was added in the 500 ml autoclaved flask containing 100 ml activated sludge extract, 100 ml alkaline bacillus medium and 50 ul Candid B (antifungal). Alkaline bacillus medium contained 1gm peptone, 0.5gm yeast extract, 1g glucose, 0.1g K₂HPo₄ and 1g Na₂CO₃. Peptone and yeast extract were autoclaved at 15psi while glucose, K₂HPo₄ and Na₂CO₃ were autoclaved at 10psi. After autoclaving the different ingredients at different psi, all the ingredients are mixed together aseptically. The enrichment flask was kept at 120 rpm for 96 hour at 35°C. For the preparation of activated sludge extract, 1Kg activated sludge was taken and dried at 50°C for 2 hour. 400g of dried activated sludge was dissolved in 960 ml single distilled water and autoclaved at 15 lbs for 1 hour. After autoclaving, the sample was centrifuged at 5000rpm for 10 minutes. The supernatant (extract) was collected and stored in sterile bottle for preparation of enrichment flask and further use. Enriched activated sludge sample was serially diluted in 0.85% saline. 100ul from each respective dilution was spread onto agar petri plates containing activated sludge extract and 50% ABM. AMB contained peptone, yeast extract, glucose, K₂HPo₄, Na₂CO₃ and 2% agar. Peptone and yeast extract were autoclaved at 15psi while glucose, K₂HPo₄ and Na₂Co₃ were autoelaved at 10psi. After autoclaving different ingredients at different psi, all ingredients are mixed together aseptically. Plates thus obtained were incubated at 35±2°C for 24-96 hrs in inverted position.

On the basis of colony morphology and color, total 2 bacterial isolates were selected to check their capability for.neutralizing the alkaline wastewater. The single isolated colonies were picked and streaked on fresh plates containing the same medium. The above step was repeated till pure colonies were obtained.

### Example 2

In order to explore the potential bacteria for neutralization of alkaline beverage industrial wastewater, total two bacteria were isolated from the pipe through which beverage industrial wastewater has been passed over a period of long time. Theses bacterial isolates were selected to check their capability for neutralizing the alkaline waste water. The single isolated colonies were picked and streaked on fresh plates containing the same medium. The above step was repeated till pure colonies were obtained. To check neutralizing capability of two isolated bacteria, 200 ml beverage industrial wastewater of high pH (12.00) was taken in 500ml glass flask at two places and each bacterial growth was added individually. Decrease in pH was monitored by pH meter (Table 1). Out of two, only one isolate was found capable to grow on high pH (12.00) and bring down the pH of wastewater within a short period of 2 hour. This bacterium was identified as Exiguobacterium *sp.* (DSM ID 03-501) and the main characteristic features are:

*Exiguobacterium sp*. (DSM ID 03-501), is facultative aerobic in nature, gram positive, is non-motile, is oxidase negative, shows optimum growth at 35°C, is capable of growth at high pH environment (pH 12.00), is capable of hydrolyzing starch and produces acids from glycerol, cellobiose, D-mannose, mannitol, methyl α -D-glucoside, amygdalin and arbutin.

### Example 3

In order to explore the potential bacteria for neutralization of alkaline beverage industrial wastewater, total two bacteria were isolated from the activated sludge of a beverage industrial ETP. Theses bacterial isolates were selected to check their capability for neutralizing the alkaline waste water. The single isolated colonies were picked and streaked on fresh plates containing the same medium. The above step was repeated till pure colonies were obtained. To check the neutralizing capability of the two isolated bacteria, 200 ml beverage industrial wastewater of high pH (12.00) was taken in 500ml glass flask at two places and each bacterial growth was added individually. Decrease in pH was monitored by a pH meter (Table 1).

**Table 1. pH reduction of alkaline wastewater by isolated alkaliphilic bacteria**

| **Bacterial Isolates** | **Reduction in pH of waste water during course of time** | | | | |
|---|---|---|---|---|---|
| | 0hr | 0.5hr | 1.0hr | 1.5hr | 2.0hr |
| Isolate 1 | 12.10 | 12.10 | 12.00 | 11.87 | 11.66 |
| **Isolate 2** (*Exiguobacterium sp)* | 12.10 | 8.33 | 7.10 | 7.06 | 7.04 |

### Example 4

In order to observe the growth of screened bacterium, *Exiguobacterium sp.* on a suitable medium, two loops from agar plate of Exiguobacterium *sp.* were streaked onto plates of NB (Nutrient broth) medium and ABM. Alkaline bacillus medium contained 1gm peptone, 0.5gm yeast extract, 1g glucose, 0.1g K₂HPo₄ and 1g Na₂CO₃. Peptone and yeast extract were autoclaved at 15psi while glucose, K₂HPo₄ and Na₂CO₃ were autoclaved at 10psi. After autoclaving the different ingredients at different psi, all the ingredients are mixed together aseptically. The plates thus obtained were incubated at 35 ± 2 ⁰C for 24 - 96 hrs in inverted position.

NB medium containing 2% agar was having original pH about 7 while ABM medium was having original pH 10.5. For increasing the pH of media, Tris-HCL and Na₂CO₃ - NaHCO₃ buffer were used.

**Table 2: growth of Exiguobacterium sp. on NB and ABM medium**

| **pH values** | **NB medium** | **ABM medium** |
|---|---|---|
| 7.00 | + | + |
| 8.00 | + | ++ |
| 9.00 | ++ | +++ |
| 10.00 | - | ++++ |
| 11.00 | - | ++++ |
| 12.00 | - | +++++ |

| | | |
|---|---|---|
| + Very poor growth; + + Poor growth; +++ Good growth; ++++ Very good growth | | |

It was observed that ABM medium of high pH is a suitable medium to grow the *Exiguobacterium sp.*

### Example 5

Alkaline beverage wastewater was neutralized with with lyophilized powder of *Exiguobacterium sp.* Bacterial pellet of 40 ml culture (O.D. = 2.00) was lyophilized and added to 500ml flask containing 200 ml alkaline beverage wastewater. Inoculated flask was kept at 35°C for one hr. Decrease in pH was observed within one hour (Table 3.)

**Table 3. pH reduction of alkaline wastewater by lyophilized bacterial powder of Exiguobacterium sp.**

| Bacterium | Reduction in pH of waste water during course of time | | | | |
|---|---|---|---|---|---|
| | 0hr | 0.5hr | 1.0hr | 1.5hr | 2.0hr |
| *Exiguobacterium sp.*(Set 1) | 12.10 | 8.43 | 7.09 | 7.04 | 7.03 |
| *Exiguobacterium sp.* (Set 2) | 12.10 | 8.77 | 7.05 | 7.03 | 7.01 |

### Advantages

1. The neutralization of alkaline beverage wastewater using, Exiguobacterium *sp.* is an economical and effective process. In conventional acid-neutralization process, tones of acid are used for the neutralization while in the developed biological process decrease the cost drastically.
2. The neutralization of alkaline beverage wastewater by biological mean is quite safe process as the utilization of acid in large quantities for the neutralization of wastewaters is not safe for the industry as the strong acid has dangerous effect on the health of workers as well as on the industrial processes. Besides this, use of large quantity of acid also increases the volume of industrial wastewaters to be drained out in the main stream.

## Claims

1. A bacterial isolate *Exiguobacterium sp.* MTCC 5183 deposited at International Depository at IMTECH, Sector 39A, Chandigarh, India recognized by Budapest Treaty.

2. A process for preparing a bacterium isolate of *Exiguobacterium sp.* MTCC 5183 useful in neutralization of alkaline waste water of beverage industry having a pH in the range of 12.00-11.5, deposited at International Depository at IMTECH, Sector 39A, Chandigarh, India recognized by Budapest Treaty, the process comprising the steps of:
(a) enriching activated sludge contaminated with bacteria comprising *Exiguobacterium sp.* MTCC 5183 by providing an autoclaved aqueous extract of dried activated sludge in an alkaline bacillus medium;
(b) culturing the bacteria;
(c) isolating the bacteria by centrifuging the culture obtained from step (b) after attaining the desired growth to obtain a pellet of bacterial cells as the bacterium isolate.

3. A process as claimed in claim 2 wherein the contaminated activated sludge is obtained from the pipe of an effluent treatment plant of a beverage industry located in Gaziabad, India.

4. A process as claimed in claim 2 wherein enrichment of the sludge is effected for a period of 40-48 hours at 100-120rpm at 33-35°C in ratio of activated sludge to medium in the range of 1:5-1:10.

5. A process as claimed in claim 2 wherein the culturing of the bacteria is carried out in the alkaline bacillus medium and at a pH in the range of 11.00 - 12.00 and at 37 to 34 °C.

6. A process as claimed in claim 2 wherein the bacteria is isolated by centrifuging the culture obtained from step (b) after attaining a growth evidenced by an optical density in the range of 1.5-2.0.

7. A process as claimed in claim 2 wherein the enrichment of the sludge is carried out by taking 5-7 g of fresh activated sludge in an autoclaved flask containing 100-110 ml autoclaved aqueous extract of dried activated sludge, 50µl Candid B anti fungal and alkaline bacillus medium.

8. A process as claimed in claim 2 wherein the autoclaved aqueous extract of dried activated sludge is prepared by centrifuging a sterilized sludge mixture at about 4000 rpm for about 20 min.

9. A process as claimed in claim 8 wherein the sterilized sludge mixture is prepared by dissolving dried activated sludge in distilled water in ratio ranges between 1:5-1:10 and autoclaving at about 15 psi for about one hour.

10. A process as claimed in claim 2 wherein the alkaline bacillus medium contains peptone, yeast extract, glucose, K₂HPO₄ and Na₂CO₃ in ratio about 1.0:0.5:1.0:0.1:1.0 by W/ V.

11. A process as claimed in claim 2 wherein the ratio between the autoclaved aqueous extract of dried activated sludge and the alkaline bacillus medium is 1:5-1:10.

12. A process as claimed in claim 2 wherein the bacterium is cultured in the alkaline bacillus medium followed by incubation at 32-37°C/80-120 rpm for 8 hours.

13. A process as claimed in claim 2 wherein the grown culture is centrifuged after attaining the heavy growth evidenced by optical density in the range of 2 -2.5.

14. A process as claimed in claim 2 further comprising the step of:
(d) dissolving the pellet obtained from step (c) in phosphate buffer.

15. A method of neutralizing alkaline wastewater of beverage industry having pH in the range of 12.00-11.5, the method comprising preparing a bacterium isolate of *Exiguobacterium sp.* MTCC 5183 by a process comprising steps (a) to (d) according to claim 14, and adding the bacterial pellet obtained from step (d) to the alkaline wastewater.

16. A method of neutralizing alkaline wastewater of beverage industry by adding a bacterial isolate according to claim 1 to the alkaline wastewater.

17. A method according to claim 16 wherein the alkaline wastewater has a pH of 12.0 to 11.5.

18. A method according to claim 17 wherein the bacterial isolate is added to the alkaline wastewater in a ratio ranging from 1:5-1: 10.

## Patentansprüche

1. Bakterielles Isolat *Exiguobacterium sp.* MTCC 5183, das bei der internationalen Hinterlegungsstelle bei IMTECH, Abteilung 39A, Chandigarh, Indien, die vom Budapester Vertrag anerkannt ist, hinterlegt worden ist.

2. Verfahren für die Herstellung eines Bakterienisolats von *Exiguobacterium sp.* MTCC 5183, das zum Neutralisieren von alkalischem Abwasser der Getränkeindustrie, das einen pH-Wert im Bereich von 12,00 - 11,5 aufweist, nützlich und bei der internationalen Hinterlegungsstelle bei IMTECH, Abteilung 39A, Chandigarh, Indien, die vom Budapester Vertrag anerkannt ist, hinterlegt worden ist, wobei das Verfahren folgende Schritte umfasst:
(a) das Anreichern von Belebtschlamm, der mit Bakterien kontaminiert ist, die *Exiguobacterium sp.* MTCC 5183 umfassen, durch Bereitstellen eines autoklavierten wässrigen Extrakts von getrocknetem Belebtschlamm in einem alkalischen Bacillusmedium;
(b) das Züchten der Bakterien;
(c) das Isolieren der Bakterien durch Zentrifugieren der aus Schritt (b) erhaltenen Kultur nach Erreichen des erwünschten Wachstums, um ein Kügelchen von Bakterienzellen als Bakteriumisolat zu erhalten.

3. Verfahren nach Anspruch 2, wobei der kontaminierte Belebtschlamm aus dem Rohr einer Abwasseraufbereitungsanlage einer Getränkeindustrie erhalten wird, die sich in Gaziabad, Indien, befindet.

4. Verfahren nach Anspruch 2, wobei die Anreicherung des Schlamms während einer Zeitspanne von 40-48 Stunden bei 100-120 UpM bei 33-35 °C in einem Verhältnis von Belebtschlamm zu Medium im Bereich von 1:5-1:10 bewirkt wird.

5. Verfahren nach Anspruch 2, wobei das Züchten der Bakterien in dem alkalischen Bacillusmedium und bei einem pH-Wert im Bereich von 11,0 - 12,00 und bei 37 bis 34 °C durchgeführt wird.

6. Verfahren nach Anspruch 2, wobei die Bakterien durch Zentrifugieren der aus Schritt (b) erhaltenen Kultur nach Erreichen eines Wachstums, das durch eine optische Dichte im Bereich von 1,5-2,0 bezeugt wird, isoliert werden.

7. Verfahren nach Anspruch 2, wobei die Anreicherung des Schlamms durch Abnehmen von 5-7 g frischem Belebtschlamm in einem autoklavierten Kolben, der 100-110 ml autoklavierten wässrigen Extrakt von getrocknetem Belebtschlamm, 50 µl Antimykotikum Candid B und alkalisches Bazillusmedium enthält, durchgeführt wird.

8. Verfahren nach Anspruch 2, wobei der autoklavierte wässrige Extrakt von getrocknetem Belebtschlamm durch etwa 20 min langes Zentrifugieren einer sterilisierten Schlammmischung bei etwa 4000 UpM hergestellt wird.

9. Verfahren nach Anspruch 8, wobei die sterilisierte Schlammmischung durch Lösen von getrocknetem Belebtschlamm in destilliertem Wasser in Verhältnisbereichen zwischen 1:5-1:10 und etwa eine Stunde langes Autoklavieren bei etwa 15 psi hergestellt wird.

10. Verfahren nach Anspruch 2, wobei das alkalische Bacillusmedium Pepton, Hefeextrakt, Glucose, K₂HOP₄ und Na₂CO₃ im Verhältnis von etwa 1,0:0,5:1,0:0,1:1,0 auf das Gew./Vol. bezogen, enthält.

11. Verfahren nach Anspruch 2, wobei das Verhältnis zwischen dem autoklavierten wässrigen Extrakt von getrocknetem Belebtschlamm und dem alkalischen Bacillusmedium 1:5-1:10 beträgt.

12. Verfahren nach Anspruch 2, wobei das Bakterium in dem alkalischen Bacillusmedium gezüchtet wird, gefolgt vom 8 Stunden langen Inkubieren bei 32-37 °C/80-120 UpM.

13. Verfahren nach Anspruch 2, wobei die gezüchtete Kultur nach Erreichen des starken Wachstums, das durch eine optische Dichte im Bereich von 2-2,5 bezeugt wird, zentrifugiert wird.

14. Verfahren nach Anspruch 2, des Weiteren den Schritt umfassend des:
(d) Lösens des aus Schritt (c) erhaltenen Kügelchens in Phosphatpuffer.

15. Verfahren zum Neutralisieren von alkalischen Abwasser der Getränkeindustrie, das einen pH-Wert im Bereich von 12,00-11,5 aufweist, wobei das Verfahren das Herstellen eines Bakteriumisolats von *Exiguobacterium sp.* MTCC 5183 durch ein Verfahren, das die Schritte (a) bis (d) nach Anspruch 14 umfasst und Zugeben des aus Schritt (d) erhaltenen Bakterienkügelchens zu dem alkalischen Abwasser umfasst.

16. Verfahren zum Neutralisieren von alkalischem Abwasser der Getränkeindustrie, durch Zugeben eines Bakterienisolats nach Anspruch 1 zu dem alkalischen Abwasser.

17. Verfahren nach Anspruch 16, wobei das alkalische Abwasser einen pH-Wert von 12,0 bis 11,5 aufweist.

18. Verfahren nach Anspruch 17, wobei das Bakterienisolat dem alkalischen Abwasser in einem Verhältnis im Bereich von 1:5-1:10 zugegeben wird.

## Revendications

1. Isolat bactérien d'*Exiguobacterium sp.* MTCC 5183 déposé auprès de l'International Depository d'IMTECH, Secteur 39A, Chandigarh, Inde, reconnu par le Traité de Budapest.

2. Procédé de préparation d'un isolat bactérien d'*Exiguobacterium sp.* MTCC 5183 utile dans la neutralisation des eaux usées alcalines de l'industrie des boissons ayant un pH situé dans la plage de 12,00 à 11,5, déposé auprès de l'International Depository d'IMTECH, Secteur 39A, Chandigarh, Inde, reconnu par le Traité de Budapest, le procédé comprenant les étapes de:
(a) enrichissement de la boue activée contaminée par des bactéries comprenant *Exiguobacterium sp.* MTCC 5183 en fournissant un extrait aqueux autoclavé de boue activée sèche dans un milieu alcalin de bacillus;
(b) culture des bactéries;
(c) isolement des bactéries par centrifugation de la culture obtenue à partir de l'étape (b) après atteinte de la croissance souhaitée pour obtenir un culot des cellules bactériennes sous la forme d'isolat bactérien.

3. Procédé tel que revendiqué selon la revendication 2 dans lequel la boue activée contaminée est obtenue à partir de la conduite d'une usine de traitement d'effluent d'une industrie des boissons située à Gaziabad, Inde.

4. Procédé tel que revendiqué selon la revendication 2 dans lequel l'enrichissement de la boue est effectué sur une durée de 40 à 48 heures à 100 à 120 tr/min à 33 à 35°C en un rapport de la boue activée au milieu situé dans la plage de 1:5 à 1:10.

5. Procédé tel que revendiqué selon la revendication 2 dans lequel la culture des bactéries est conduite dans le milieu alcalin de bacillus et à un pH situé dans la plage de 11,00 à 12,00 et à 37 à 34°C.

6. Procédé tel que revendiqué selon la revendication 2 dans lequel les bactéries sont isolées par centrifugation de la culture obtenue à partir de l'étape (b) après atteinte d'une croissance mise en évidence par une densité optique située dans la plage de 1,5 à 2,0.

7. Procédé tel que revendiqué selon la revendication 2 dans lequel l'enrichissement de la boue est conduit en prenant 5 à 7 g de boue activée fraîche dans un flacon autoclavé contenant 100 à 110 ml d'extrait aqueux autoclavé de boue activée sèche, 50 µl d'agent antifongique Candid B et de milieu alcalin de bacillus.

8. Procédé tel que revendiqué selon la revendication 2 dans lequel l'extrait aqueux autoclavé de la boue activée sèche est préparé par centrifugation d'un mélange de boue stérilisé à environ 4 000 tr/min durant environ 20 min.

9. Procédé tel que revendiqué selon la revendication 8 dans lequel le mélange de boue stérilisé est préparé par dissolution de la boue activée sèche dans de l'eau distillée en un rapport qui s'étend entre 1:5 à 1:10 et autoclavage à environ 15 lb/po² durant environ une heure.

10. Procédé tel que revendiqué selon la revendication 2 dans lequel le milieu alcalin de bacillus contient de la peptone, de l'extrait de levure, du glucose, du K₂HPO₄ et du Na₂CO₃ en un rapport d'environ 10:0,5:1,0:0,1:1,0 en pds/vol.

11. Procédé tel que revendiqué selon la revendication 2 dans lequel le rapport entre l'extrait aqueux autoclavé de la boue activée sèche et le milieu alcalin de bacillus est 1:5 à 1:10.

12. Procédé tel que revendiqué selon la revendication 2 dans lequel la bactérie est cultivée dans le milieu alcalin de bacillus suivi de l'incubation à 32 à 37°C/80 à 120 tr/min durant 8 heures.

13. Procédé tel que revendiqué selon la revendication 2 dans lequel la culture produite est centrifugée après atteinte de la croissance vigoureuse mise en évidence par densité optique située dans la plage de 2 à 2,5.

14. Procédé tel que revendiqué selon la revendication 2 comprenant en outre l'étape de:
(d) dissolution du culot obtenu à partir de l'étape (c) dans du tampon phosphate.

15. Procédé de neutralisation des eaux usées alcalines de l'industrie des boissons ayant un pH situé dans la plage de 12,00 à 11,5, le procédé comprenant la préparation d'un isolat bactérien d'*Exiguobacterium sp.* MTCC 5183 par un procédé comprenant les étapes (a) à (d) selon la revendication 14, et l'addition du culot bactérien obtenu à partir de l'étape (d) aux eaux usées alcalines.

16. Procédé de neutralisation des eaux usées alcalines de l'industrie des boissons par addition d'un isolat bactérien selon la revendication 1 aux eaux usées alcalines.

17. Procédé selon la revendication 16 dans lequel les eaux usées alcalines ont un pH de 12,0 à 11,5.

18. Procédé selon la revendication 17 dans lequel l'isolat bactérien est ajouté aux eaux usées alcalines en un rapport s'étendant de 1:5 à 1:10.
